# EUROPEAN PATENT SPECIFICATION

(11) **EP 4 514 459 B1**
(45) Date of publication and mention of the grant of the patent: **11.02.2026**
(21) Application number: 23717583.1
(22) Date of filing: 18.04.2023
(51) Int. Cl.: A61N 1/37, A61N 1/39

(54) **METHOD FOR CONTROLLING A MEDICAL DEVICE IMPLANTED IN A PATIENT IN PRESENCE OF AN EXTERNAL DEVICE IN THE PATIENT'S ENVIRONMENT**
VERFAHREN ZUM STEUERN EINER IN EINEM PATIENTEN IMPLANTIERTEN MEDIZINISCHEN VORRICHTUNG BEI VORHANDENSEIN EINER EXTERNEN VORRICHTUNG IN DER UMGEBUNG DES PATIENTEN
PROCÉDÉ DE COMMANDE D'UN DISPOSITIF MÉDICAL IMPLANTÉ DANS UN PATIENT EN PRÉSENCE D'UN DISPOSITIF EXTERNE DANS L'ENVIRONNEMENT DU PATIENT

(30) Priority: 28.04.2022 EP 22170528
(43) Date of publication of application: 05.03.2025
(73) Proprietor: BIOTRONIK SE & Co. KG, 12359 Berlin (DE)
(72) Inventor: DOERR, Thomas, 12437 Berlin (DE); LANG, Volker, 12161 Berlin (DE); DIEM, Bjoern Henrik, 14197 Berlin (DE)
(74) Representative: Biotronik Corporate Services SE
(86) International application number: PCT/EP2023/059976
(87) International publication number: WO 2023/208643

(56) References cited:
- US-A1- 2008 147 135
- US-A1- 2011 148 400
- US-A1- 2011 152 667
- US-A1- 2013 289 638
- US-B2- 8 929 995

## Description

The present invention relates to a computer-implemented method for controlling a medical device, which is implanted in a patient, in presence of an external device in the patient's environment. Furthermore, the invention relates to a control unit, a computer program and a computer-readable medium for carrying out the method and to an implantable medical device comprising said control unit.

The presence of an external device in the environment of a patient carrying an implantable medical device may cause undesirable electromagnetic interference. This may have a negative impact on diagnostic and/or therapeutic functions of the implantable medical device and, in some cases, may even lead to the patient no longer being able to work in certain environments.

It is further known to control a machine based on information which is exchanged between the machine and an implantable medical device.

For example, US 2011/0092802 A1 describes a control device for controlling a machine, e.g., a vehicle, based on data received from an implant in dependence on the fitness of the implant wearer.

US 2013/0289638 A1 describes an implantable medical device (IMD) that automatically detects the presence of an external magnetic field, such as that generated by an MRI device. The IMD includes a torque sensor configured to generate an output signal that varies as a function of a torque imposed on the torque sensor by an external magnetic field and a control module configured to control operation of the implantable medical device based on the signal output of the torque sensor.

US 8,929,995 B2 discloses an implantable medical device that may include a telemetry module, a sensing module, a therapy delivery module, and a processor. The processor may be configured to detect a patient event based on data generated by the sensing module, operate the IMD in a first mode in which the telemetry module is disabled and the therapy delivery module is at least partially disabled when the patient event is not detected, and operate the IMD in a second mode in which the telemetry module is enabled and the therapy delivery module is at least partially disabled when the patient event is detected. In some examples, the processor is configured to, in the second mode, generate a notification of the cardiac arrhythmia and transmit the notification to an external device via the telemetry module. The external device may reside inside an MRI room or outside the MRI room, and may communicate with other devices.

US 2011/0148400 A1 describes a method that allows the specificity of an automatic MRT detection to be increased in a simple manner. This is achieved using an automatically calibrating position sensor, so that the user does not have to perform additional calibration of this sensor. Incorrect sensor calibrations are thus eliminated as well.

US 2011/0152667 A1 teaches a device and a method for detecting electromagnetic fields, in particular, fields occurring in magnetic resonance tomography (MRT) or magnetic resonance imaging (MRI) tests. An implantable medical device (IMD), contained in a hermetically sealed housing, includes a control unit, a programming coil, and a communication unit, wherein the communication unit, together with the programming coil, is designed to allow communication between an external programming device and the IMD by utilizing alternating electromagnetic fields, and further comprising a detection unit for MRT interference fields, characterized in that the detection unit is designed in such a way that voltage profiles induced in the programming coil and originating from a pulsed alternating electromagnetic field of the MRT (gradient field) are detected, and a corresponding MRT detection signal is transmitted from the detection unit to the control unit, if communication with a programming device is not detected at the same time.

US 2008/0147135 A1 describes an implantable medical device (IMD) which includes an input filter circuit having variable capacitor elements that can be electronically adjusted based upon current operating conditions (such as electromagnetic conditions, noise conditions, and/or environmental conditions). The variable capacitor elements can be adjusted to accommodate pre-designated operating modes of the IMD and/or dynamically in response to changing operating conditions. In one embodiment, the variable capacitor elements are realized using digitally programmable switched capacitor arrangements.

It may be seen as an objective of the present invention to provide an improved method for controlling a medical device, which is implanted in a patient, in presence of an external device in the patient's environment. Another objective of the invention may be to provide a control unit, a computer program and a computer-readable medium for carrying out said method as well as an implantable medical device comprising said control unit.

These objectives may be achieved by the subject-matter of the independent claims. Advantageous embodiments are defined in the dependent claims as well as in the corresponding specification and figures.

A first aspect of the invention relates to a computer-implemented method for controlling a medical device, which is implanted in a patient, in presence of an external device in the patient's environment. The method comprises at least the following steps: receiving a detection signal indicating the presence of the external device in the patient's environment, wherein the detection signal comprises a unique identifier for the external device; selecting an operating mode for the medical device by comparing the identifier to a list of known identifiers, wherein each known identifier is associated with a predefined operating mode for the medical device; and applying the selected operating mode to the medical device, thereby adjusting the medical device so that undesirable electromagnetic interaction between the medical device and the external device is reduced.

The method may be carried out automatically by a processor.

Electromagnetic interaction, also referred to as electromagnetic interference (EMI), may be understood as a disturbance generated by the external device that may affect electrical and/or electronic components of the medical device by electromagnetic induction, electrostatic coupling and/or conduction (and *vice versa).* The disturbance, which may be caused by changing electrical currents and voltages, may degrade the performance of these components or even stop them from functioning.

The detection signal may be seen as an information about a spatial proximity of the external device with respect to the medical device. The detection signal may have been generated by and sent from the external device, e.g., one or more detection units of the external device, to the medical device. Such a detection unit may be a transmitter, e.g., a radio beacon, or a transceiver, e.g., an active or passive transponder. In other words, the detection signal may be received via a unidirectional or bidirectional communication link. The detection signal may be received via a wireless communication link and/or the Internet. The detection signal may be received directly from the external device or indirectly via at least one additional data communication device (e.g., a smartphone, smartwatch, tablet, laptop, PC or server) which interconnects the medical device and the external device for data communication.

The identifier may be seen as a unique predetermined code associated with one external device or with one group of identical or similar external devices. Thus, each known identifier in the list may stand for one (known) external device or one group of (known) identical or similar external devices.

The list of known identifiers may be stored in a memory of the medical device. For example, the list may comprise more than 10, in particular more than 100 or even more than 1,000 or more than 10,000 known identifiers.

Each predefined operating mode may be defined by a set of specific operating parameters, wherein different predefined operating modes in the list may differ from each other in at least one of these parameters.

Different known identifiers in the list may be associated with different or identical predefined operating modes.

The selected operating mode may be selected by searching in the list of known identifiers for an item which is identical to the identifier encoded by the detection signal and by selecting the predefined operating mode associated with this item.

The method helps to ensure safe operation of active implants in the presence of external devices, such as machines, industrial plants or vehicles, especially in professional environments, in an efficient and reliable manner without the need of manual interaction. Thus, possible limitations for patients carrying active implants and working in potentially hostile environments, e.g., in the vicinity of a powerful electric machine, can be at least partially eliminated.

A second aspect of the invention relates to a control unit comprising a processor configured to carry out the method as described above and below. The control unit may include hardware and/or software modules. In addition to the processor, the control unit may include a memory and data communication interfaces for data communication with peripheral devices, e.g., an external device as described above and below.

The control unit may be part of an implantable medical device. However, it is also possible that the control unit is integrated in a user device (e.g., a remote, smartphone, smartwatch, tablet, laptop or PC) connected to the implantable medical device for data communication. For example, the (external) user device may be configured to run an application for controlling one or more functions of the implantable medical device remotely.

A third aspect of the invention relates to an implantable medical device comprising the control unit as described above and below. The implantable medical device may be, for example, an implantable pulse generator (IPG), an implantable cardioverter defibrillator (ICD), a cardiac resynchronization therapy (CRT) pacemaker, a neurostimulator, an electrocardiogram (ECG) recorder, a pressure sensor, a biochemical sensor, a drug pump or a hearing aid.

Further aspects of the invention relate to a computer program comprising instructions which, when the program is executed by a processor, cause the processor to carry out the method as described above and below and to a computer-readable medium in which the computer program is stored. The computer program may be executed by a processor of the control unit.

The computer-readable medium may be a volatile or non-volatile data storage device. For example, the computer-readable medium may be a hard drive, USB (universal serial bus) storage device, RAM (random-access memory), ROM (read-only memory), EPROM (erasable programmable read-only memory) or flash memory. The computer-readable medium may also be a data communication network for downloading program code, such as the Internet or a data cloud.

It should be noted that features of the method as described above and below may be features of the control unit, the computer program and the computer-readable medium, and *vice versa.*

Embodiments of the invention may be considered, without limiting the invention, as being based on the ideas and findings described below.

According to an embodiment, the list may comprise known identifiers for different device types. Additionally or alternatively, the list may comprise known identifiers for at least one of the following device types: an industrial machine, an industrial tool, an industrial robot, a vehicle (e.g., a car, truck, bus or motorcycle), in particular an electric vehicle, a security gate, a further medical device (e.g., an MRI system). This allows the medical device to be automatically adapted to (completely) different device types without the need of reprograming the medical device.

According to an embodiment, by applying the selected operating mode, at least one diagnostic and/or therapeutic function of the medical device may be modified or deactivated. Such a function may be configured, for example, to provide electric pulses in a controlled manner, e.g., in order to stimulate the patient's heart, spine or brain, and/or to monitor (electrical) biosignals from the patient. Thus, modifying the function(s) may comprise, for example, modifying the form and/or duration of these electric pulses and/or modifying one or more filters for filtering the biosignals. This embodiment may significantly reduce the risk of EMI-related inaccuracies, or even malfunctions, when operating the medical device near the external device.

The medical device may be configured to sense biosignals from the patient along different sensing vectors. According to an embodiment, applying the selected operating mode may cause the medical device to switch between these sensing vectors, for example, from one or more currently active sensing vectors to one or more alternative sensing vectors which may be less susceptible to EMI in the presence of the external device. For example, each sensing vector may be defined by a pair of electrodes in contact with the patient's body in dependence on their position relative to the patient's body and their mutual distance. Such an electrode may be, for example, part of an implantable lead or implantable housing of the medical device. This makes it possible to reduce EMI without having to interrupt or completely deactivate sensing of the biosignals.

According to an embodiment, by applying the selected operating mode, at least one hardware component of the medical device, in particular at least one sensor of the medical device, may be deactivated. This may be done, for example, by disconnecting the hardware component(s) from a battery of the medical device or by ignoring an output signal provided by the hardware component(s). The sensor(s) may be configured to sense (electrical) biosignals from the patient and/or magnetic fields.

The medical device may comprise an interference detector configured to detect interference in an output signal provided by at least one sensor of the medical device. The sensor(s) may be configured to sense (electrical) biosignals from the patient and/or magnetic fields. According to an embodiment, by applying the selected operating mode, the interference detector may be modified or deactivated. This may significantly reduce inaccuracies and/or malfunctions of the interference detector caused by EMI, in particular in the presence of very powerful electrical machines such as electric vehicles or industrial robots.

According to an embodiment, applying the selected operating mode may prevent the medical device from switching into a power saving mode. This ensures that the medical device is fully functional in the presence of the external device.

The medical device may comprise a recording function configured to record an output signal provided by at least one sensor of the medical device. The sensor(s) may be configured to sense (electrical) biosignals from the patient and/or magnetic fields. According to an embodiment, applying the selected operating mode may cause the recording function to:
- start recording the output signal and/or mark new recordings of the output signal as being recorded in presence of the external device; or
- stop recording the output signal.

For example, the (new) recordings may be used to identify undesirable electromagnetic interference in the presence of the external device. Such information may be used to further improve the predefined operating modes and/or to define new operating modes for the medical device. Deactivating the recording function may help to further reduce undesirable electromagnetic interference.

According to an embodiment, the selected operating mode may be applied only as long as the detection signal is received. In other words, the medical device may automatically be switched from the selected operating mode back into a previous operating mode when the detection signal is no longer received, e.g., when a period of time since the detection signal has been last received is longer than 1 s, 10 s or 1 min. This enables automatic activation and deactivation of the corresponding predefined operating mode without additional interaction, e.g., by the patient or a physician.

According to an embodiment, the detection signal may be received via a wireless data communication link for data communication between the medical device and the external device, in particular unidirectional data communication. For example, the detection signal may be received via Bluetooth (e.g., Bluetooth Low Energy), Wi-Fi, LoRaWAN (low-power wide-area network), WPAN, ZigBee, ISM (industrial, scientific and medical radio), MICS (Medical Implant Communication Service), a cellular network (e.g., GSM, LTE or 5G), near-field communication (NFC), ultra-wideband or a combination of at least two of these examples.

It may be that the detection signal is transmitted in regular time intervals by a transmitter or transceiver of the external device within a specific range of, for example, 100 m or less, 10 m or less or 1 m or less. A receiver or transceiver integrated in the medical device may then receive the detection signal as soon as the patient carrying the medical device is within said range. This has the advantage that no first (interrogating) signal has to be sent from the medical device to the external device prior to receiving the detection signal. This may improve energy efficiency of the medical device and reduce manufacturing costs.

Alternatively, the method may comprise a step of sending a first signal from the medical device to the external device, wherein the first signal may cause the external device to send the detection signal as a second signal.

According to an embodiment, the detection signal may be received via Bluetooth, for example, using Bluetooth Low Energy (BLE) technology. This enables efficient and reliable short-range data communication between the medical device and the external device.

It should be noted that possible features and advantages of embodiments of the invention are described above and below partly with reference to a method for controlling an implantable medical device and partly with reference to a control unit specifically designed for carrying out said method. A person skilled in the art will recognize that the features described for individual embodiments may be suitably transferred to other embodiments in an analogous manner, may be adapted and/or interchanged to arrive at further embodiments of the invention and possibly synergistic effects.

Advantageous embodiments of the invention are further explained below with reference to the accompanying drawings. Neither the drawings nor the description are to be construed as limiting the invention.
- Fig. 1: shows a patient carrying an implantable medical device according to an embodiment of the invention in the vicinity of an industrial robot.
- Fig. 2: shows the implantable medical device of Fig. 1 in more detail.

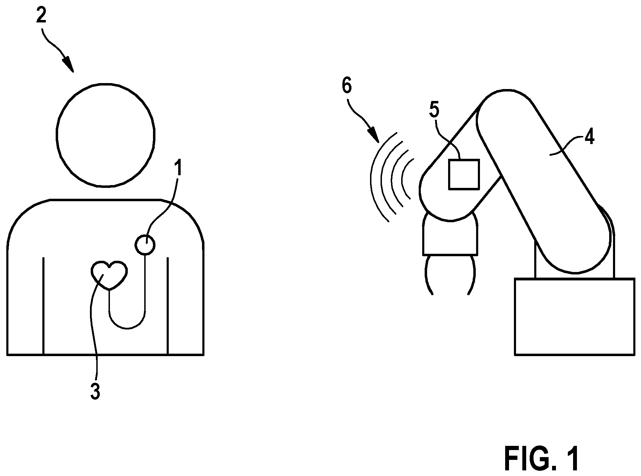

Fig. 1 shows a medical device 1 implanted in the body of a patient 2. For example, the medical device 1 may be adapted to generate electrical signals for stimulating the patient's heart 3 and/or to monitor electrical biosignals from the patient's heart 3. The patient 2 is near an external device 4, here an industrial robot.

The medical device 1 and the external device 4 may be interconnected for data communication via a wireless communication link (unidirectional or bidirectional), for example, via Bluetooth. However, all low-power communication methods are, in principle, suitable for data communication between the two devices 1, 4.

The external device 4 may comprise one or more detection units 5, e.g., one or more radio beacons (for unidirectional data communication) and/or one or more active and/or passive transponders (for bidirectional data communication), for transmitting via the wireless communication link a detection signal 6 which encodes a unique identifier for the external device 4. For example, the detection unit 5 may be a Bluetooth low energy (BLE) beacon in the form of an asset tag attached to the external device 4, here a moving arm of the industrial robot.

In addition, the medical device 1 may be configured for encrypted data communication with the external device 4.

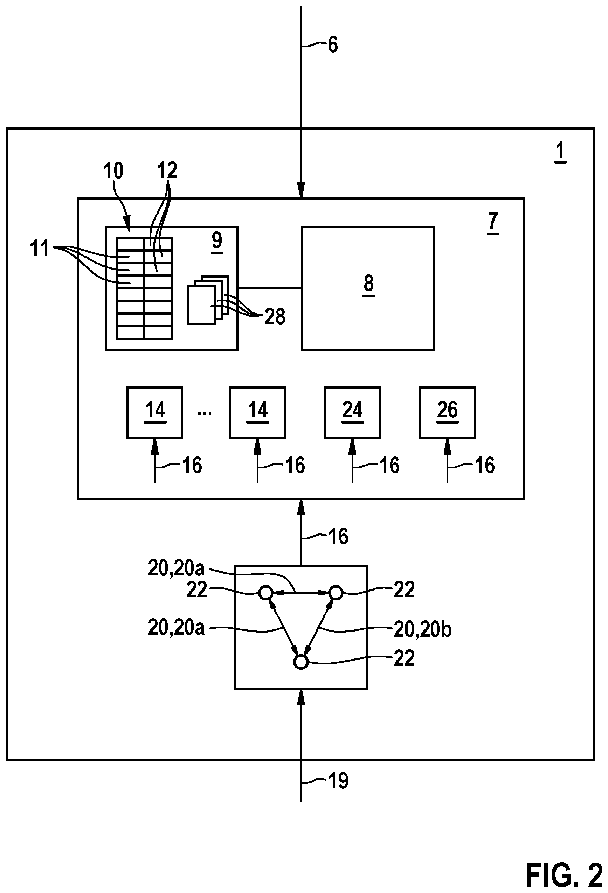

As shown in Fig. 2, the medical device 1 may comprise a control unit 7 with a processor 8 and a memory 9. The processor 8 may be configured to carry out a method for controlling the medical device 1 by executing instructions of a computer program which may be stored in the memory 9. The method is described in more detail below.

In a first step, the control unit 7 receives the detection signal 6. This may be the case when the patient 2, and thus the control unit 7, is within the range of the detection unit 5, for example, when the distance of the control unit 7 from the detection unit 5 is 10 m or less or 5 m or less.

As shown here, the control unit 7 may receive the detection signal 6 directly from the detection unit 5.

However, it is also possible for the detection signal 6 to be transmitted via at least one additional relay device which connects the medical device 1 to the external device 4 for data communication. The additional relay device may be, for example, a cloud or network instance which allows data communication only with valid authentication of the medical device 1 and/or the external device 4. This further increases data security.

In a second step, the control unit 7 selects an operating mode for the medical device 1 based on the detection signal 6. This may be done by comparing the identifier encoded by the detection signal 6 with a list 10 of known identifiers 11, wherein each known identifier 11 is associated with a predefined operating mode 12 for the medical device 1. The list 10 may be stored in the memory 9. The selected operating mode 12 may be a predefined operating mode 12 whose known identifier 11 is identical to the identifier encoded by the detection signal 6.

In a third step, the control unit 7 applies the selected operating mode 12 to the medical device 1, thereby changing one or more operating parameters of the medical device 1 in such a manner that undesirable electromagnetic interference between the medical device 1 and the external device 4 is avoided or at least reduced. The selected operating mode 12 may have been individually adapted to the external device 4 by programming the medical device 1 accordingly.

The method may additionally comprise a fourth step of switching the medical device 1 from the selected operating mode 12 back to a previous operating mode when the detection signal 6 is no longer received, e.g., when a period of time since the detection signal 6 has been last received is longer than 1 s, 10 s or 1 min.

For example, the list 10 may comprise known identifiers 11 for different industrial robots.

It is also possible that the list 10 comprises known identifiers 11 for different types of external devices 4, such as industrial robots, industrial machines, industrial tools, (electric) vehicles, security gates or further medical devices (e.g., MRI systems).

For example, the control unit 7 may comprise one or more diagnostic and/or therapeutic functions 14 which may be configured to process an output signal 16 of one or more sensors 18 in contact with the patient's body and to control one or more active elements of the medical device 1 accordingly (the active elements may include the sensor(s) 18). The function(s) 14, as well as other functions of the medical device 1 (see below), may be software and/or hardware modules of the control unit 7.

By applying the selected operating mode 12, one or more diagnostic and/or therapeutic functions 14 may be modified or deactivated and, additionally, one or more other diagnostic and/or therapeutic functions 14 may be activated.

The output signal 16 may encode an electrical biosignal 19 received from the patient 2 as input signal, wherein the biosignal 19 may have been sensed by the sensor(s) 18 along different sensing vectors 20. The position, orientation and length of each sensing vector 20 may be defined by a pair of electrodes 22 which are in contact with different portions of the patient's body.

Additionally or alternatively, the sensor(s) 18 may be adapted to sense magnetic fields in the patient's environment.

For example, by applying the selected operating mode 12, at least one currently active sensing vector 20a may be blocked and instead at least one alternative sensing vector 20b less susceptible to EMI may be activated and used to sense the biosignal 19.

Alternatively, the sensor(s) 18 may be completely deactivated by applying the selected operating mode 12.

The control unit 7 may additionally comprise an interference detector 24 configured to detect interference in the output signal 16. Applying the selected operating mode 12 may modify the interference detector 24 so that certain interference patterns in the output signal 16 are temporarily ignored by the interference detector 24. Alternatively, applying the selected operating mode 12 may completely deactivate the interference detector 24.

The interference detector 24 may, for example, be configured to detect the presence of an MRI system as the external device 4 based on interference in the output signal 16, wherein the control unit 7 may be configured to switch the medical device 1 into a special operating mode when the MRI system is detected.

In addition, the control unit 7 may comprise a recording function 26 configured to record the output signal 16 and/or to store recordings 28 of the output signal 16 in the memory 9. Applying the selected operating mode 12 may cause the recording function 26 to start recording the output signal 16 and/or to mark new recordings 28 as being recorded in presence of the external device 4. Alternatively, recording of the output signal 16 may be stopped by applying the selected operating mode 12.

For example, in the case of a loop recorder (biomonitor), episode recording may be enabled or disabled, or episode recordings based on, for example, P wave detection may be temporarily suspended to avoid inadequate recordings and/or EMI.

Additionally or alternatively, applying the selected operating mode 12 may prevent the medical device 1 from switching into a power saving mode. For example, night reduction may be deactivated during shift work or in an active phase of the patient 2 during on-call duty.

In other words, the medical device 1 may automatically modify its operating mode upon detection of a registered external device 4. As pointed out above, such automatic modification may include, for example:
- modification of an interference signal detection or an interference mode of the medical device 1, for example, when a powerful electric vehicle is detected as the external device 4;
- activation and/or deactivation of at least one diagnostic and/or therapeutic function 14 of the medical device 1;
- activation and/or deactivation of at least one technical component, in particular at least one sensor 18 (e.g., a magnetic field sensor), of the medical device 1;
- activation and/or deactivation of at least one function and/or algorithm which could cause an undesirable interaction with the external device 4, for example, impair proper operation of a machine by the patient 2 (for example, in the case of an implantable pulse generator, night reduction may be deactivated).

For example, in order to detect a vehicle as the external device 4, the medical device 1 may exchange BLE pairing information with an entertainment system of the vehicle, e.g., an audio system.

It should be noted that, in the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope of the claims.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention.

## Claims

1. A computer-implemented method for controlling a medical device (1) implanted in a patient (2) in presence of an external device (4) in the patient's (2) environment, the method comprising:
receiving a detection signal (6) indicating the presence of the external device (4) in the patient's (2) environment, wherein the detection signal (6) comprises a unique identifier for the external device (4);
selecting an operating mode for the medical device (1) by comparing the identifier to a list (10) of known identifiers (11), wherein each known identifier (11) is associated with a predefined operating mode (12) for the medical device (1); and
applying the selected operating mode (12) to the medical device (1), thereby adjusting the medical device (1) so that undesirable electromagnetic interaction between the medical device (1) and the external device (4) is reduced,
wherein the list (10) comprises known identifiers (11) for different device types; and/or
wherein the list (10) comprises known identifiers (11) for at least one of the following device types: an industrial machine, an industrial tool, an industrial robot, a vehicle, a security gate, a further medical device.

2. The method of claim 1, wherein each predefined operating mode is defined by a set of specific operating parameters, wherein different predefined operating modes in the list (10) may differ from each other in at least one of these parameters.

3. The method of one of the previous claims,
wherein, by applying the selected operating mode (12), at least one diagnostic and/or therapeutic function (14) of the medical device (1) is modified or deactivated.

4. The method of one of the previous claims,
wherein the medical device (1) is configured to sense biosignals (19) from the patient (2) along different sensing vectors (20, 20a, 20b);
wherein applying the selected operating mode (12) causes the medical device (1) to switch between the sensing vectors (20, 20a, 20b).

5. The method of one of the previous claims,
wherein, by applying the selected operating mode (12), at least one hardware component (18) of the medical device (1), in particular at least one sensor (18) of the medical device (1), is deactivated.

6. The method of one of the previous claims,
wherein the medical device (1) comprises an interference detector (24) configured to detect interference in an output signal (16) provided by at least one sensor (18) of the medical device (1);
wherein, by applying the selected operating mode (12), the interference detector (24) is modified or deactivated.

7. The method of one of the previous claims,
wherein applying the selected operating mode (12) prevents the medical device (1) from switching into a power saving mode.

8. The method of one of the previous claims,
wherein the medical device (1) comprises a recording function (26) configured to record an output signal (16) provided by at least one sensor (18) of the medical device (1);
wherein applying the selected operating mode (12) causes the recording function (26) to:
- start recording the output signal (16) and/or mark new recordings (28) of the output signal (16) as being recorded in presence of the external device (4); or
- stop recording the output signal (16).

9. The method of one of the previous claims,
wherein the selected operating mode (12) is applied only as long as the detection signal (6) is received.

10. The method of one of the previous claims,
wherein the detection signal (6) is received via a wireless data communication link for data communication, in particular unidirectional data communication, between the medical device (1) and the external device (4).

11. The method of one of the previous claims,
wherein the detection signal (6) is received via Bluetooth.

12. A control unit (7) comprising a processor (8) configured to carry out the method of one of the previous claims.

13. An implantable medical device (1) comprising the control unit (7) of claim 12.

14. The implantable medical device (1) of claim 13, wherein the implantable medical device is at least one of an implantable pulse generator (IPG), an implantable cardioverter defibrillator (ICD), a cardiac resynchronization therapy (CRT) pacemaker, a neurostimulator, an electrocardiogram (ECG) recorder, a pressure sensor, a biochemical sensor, a drug pump or a hearing aid.

15. A computer program comprising instructions which, when the program is executed by a processor (8), cause the processor (8) to carry out the method of one of claims 1 to 11.

## Patentansprüche

1. Ein computergestütztes Verfahren zur Steuerung eines medizinischen Geräts (1), das in einen Patienten (2) implantiert ist, in Gegenwart eines externen Geräts (4) in der Umgebung des Patienten (2), wobei das Verfahren umfasst:
Empfangen eines Erkennungssignals (6), das die Anwesenheit des externen Geräts (4) in der Umgebung des Patienten (2) anzeigt, wobei das Erkennungssignal (6) eine eindeutige Kennung für das externe Gerät (4) umfasst;
Auswählen eines Betriebsmodus für das medizinische Gerät (1) durch Vergleichen der Kennung mit einer Liste (10) bekannter Kennungen (11), wobei jede bekannte Kennung (11) einem vordefinierten Betriebsmodus (12) für das medizinische Gerät (1) zugeordnet ist; und
Anwenden des ausgewählten Betriebsmodus (12) auf das medizinische Gerät (1), wodurch das medizinische Gerät (1) so eingestellt wird, dass unerwünschte elektromagnetische Wechselwirkungen zwischen dem medizinischen Gerät (1) und dem externen Gerät (4) reduziert werden,
wobei die Liste (10) bekannte Identifikatoren (11) für verschiedene Gerätetypen umfasst; und/oder
wobei die Liste (10) bekannte Identifikatoren (11) für mindestens einen der folgenden Gerätetypen umfasst: eine Industriemaschine, ein Industriewerkzeug, einen Industrieroboter, ein Fahrzeug, ein Sicherheitstor, ein weiteres medizinisches Gerät.

2. Verfahren nach Anspruch 1, wobei jeder vordefinierte Betriebsmodus durch einen Satz spezifischer Betriebsparameter definiert ist, wobei sich verschiedene vordefinierte Betriebsmodi in der Liste (10) in mindestens einem dieser Parameter voneinander unterscheiden können.

3. Verfahren nach einem der vorstehenden Ansprüche, wobei durch Anwenden des ausgewählten Betriebsmodus (12) mindestens eine diagnostische und/oder therapeutische Funktion (14) der medizinischen Vorrichtung (1) modifiziert oder deaktiviert wird.

4. Verfahren nach einem der vorhergehenden Ansprüche, wobei das medizinische Gerät (1) so konfiguriert ist, dass es Biosignale (19) vom Patienten (2) entlang verschiedener Erfassungsvektoren (20, 20a, 20b) erfasst;
wobei die Anwendung des ausgewählten Betriebsmodus (12) bewirkt, dass das medizinische Gerät (1) zwischen den Erfassungsvektoren (20, 20a, 20b) umschaltet.

5. Verfahren nach einem der vorstehenden Ansprüche, wobei durch Anwenden des ausgewählten Betriebsmodus (12) mindestens eine Hardwarekomponente (18) der medizinischen Vorrichtung (1), insbesondere mindestens ein Sensor (18) der medizinischen Vorrichtung (1), deaktiviert wird.

6. Verfahren nach einem der vorstehenden Ansprüche,
wobei das medizinische Gerät (1) einen Interferenzdetektor (24) umfasst, der so konfiguriert ist, dass er Interferenzen in einem Ausgangssignal (16) erkennt, das von mindestens einem Sensor (18) des medizinischen Geräts (1) bereitgestellt wird;
wobei durch Anwendung des ausgewählten Betriebsmodus (12) der Interferenzdetektor (24) modifiziert oder deaktiviert wird.

7. Verfahren nach einem der vorhergehenden Ansprüche, wobei die Anwendung des ausgewählten Betriebsmodus (12) verhindert, dass das medizinische Gerät (1) in einen Energiesparmodus wechselt.

8. Verfahren nach einem der vorhergehenden Ansprüche, wobei das medizinische Gerät (1) eine Aufzeichnungsfunktion (26) umfasst, die so konfiguriert ist, dass sie ein Ausgangssignal (16) aufzeichnet, das von mindestens einem Sensor (18) des medizinischen Geräts (1) bereitgestellt wird;
wobei das Anwenden des ausgewählten Betriebsmodus (12) bewirkt, dass die Aufzeichnungsfunktion (26):
- die Aufzeichnung des Ausgangssignals (16) zu starten und/oder neue Aufzeichnungen (28) des Ausgangssignals (16) als aufgezeichnet zu markieren, wenn das externe Gerät (4) vorhanden ist; oder
- die Aufzeichnung des Ausgangssignals (16) zu beenden.

9. Verfahren nach einem der vorhergehenden Ansprüche, wobei der ausgewählte Betriebsmodus (12) nur so lange angewendet wird, wie das Erkennungssignal (6) empfangen wird.

10. Verfahren nach einem der vorstehenden Ansprüche, wobei das Detektionssignal (6) über eine drahtlose Datenkommunikationsverbindung für die Datenkommunikation, insbesondere die unidirektionale Datenkommunikation, zwischen dem medizinischen Gerät (1) und dem externen Gerät (4) empfangen wird.

11. Verfahren nach einem der vorstehenden Ansprüche, wobei das Detektionssignal (6) über Bluetooth empfangen wird.

12. Eine Steuereinheit (7) mit einem Prozessor (8), der so konfiguriert ist, dass er das Verfahren nach einem der vorhergehenden Ansprüche ausführt.

13. Implantierbares medizinisches Gerät (1) mit der Steuereinheit (7) nach Anspruch 12.

14. Implantierbares medizinisches Gerät (1) nach Anspruch 13, wobei das implantierbare medizinische Gerät mindestens eines von einem implantierbaren Impulsgenerator (IPG), einem implantierbaren Kardioverter-Defibrillator (ICD), einem Herzresynchronisationstherapie-Schrittmacher (CRT), einem Neurostimulator, einem Elektrokardiogramm (EKG)-Rekorder, einem Drucksensor, einem biochemischen Sensor, einer Medikamentenpumpe oder einem Hörgerät ist.

15. Ein Computerprogramm, das Anweisungen umfasst, die, wenn das Programm von einem Prozessor (8) ausgeführt wird, den Prozessor (8) veranlassen, das Verfahren nach einem der Ansprüche 1 bis 11 auszuführen.

## Revendications

1. Procédé mis en œuvre par ordinateur de commande d'un dispositif médical (1) implanté chez un patient (2) en présence d'un dispositif externe (4) dans l'environnement du patient (2), le procédé comprenant les étapes consistant à :
recevoir un signal de détection (6) indiquant la présence du dispositif externe (4) dans l'environnement du patient (2), dans lequel le signal de détection (6) comprend un identifiant unique pour le dispositif externe (4) ;
sélectionner un mode de fonctionnement pour le dispositif médical (1) en comparant l'identifiant à une liste (10) d'identifiants connus (11), dans lequel chaque identifiant connu (11) est associé à un mode de fonctionnement prédéfini (12) pour le dispositif médical (1) ; et
appliquer le mode de fonctionnement sélectionné (12) au dispositif médical (1), ajustant ainsi le dispositif médical (1) de telle sorte que l'interaction électromagnétique indésirable entre le dispositif médical (1) et le dispositif externe (4) est réduite,
dans lequel la liste (10) comprend des identifiants connus (11) pour différents types de dispositif ; et/ou
dans lequel la liste (10) comprend des identifiants connus (11) pour au moins l'un des types de dispositif suivants : une machine industrielle, un outil industriel, un robot industriel, un véhicule, un portique de sécurité, un dispositif médical supplémentaire.

2. Procédé selon la revendication 1, dans lequel chaque mode de fonctionnement prédéfini est défini par un ensemble de paramètres de fonctionnement spécifiques, dans lequel différents modes de fonctionnement prédéfinis dans la liste (10) peuvent différer les uns des autres pour au moins l'un de ces paramètres.

3. Procédé selon l'une des revendications précédentes,
dans lequel, en appliquant le mode de fonctionnement sélectionné (12), au moins une fonction diagnostique et/ou thérapeutique (14) du dispositif médical (1) est modifiée ou désactivée.

4. Procédé selon l'une des revendications précédentes,
dans lequel le dispositif médical (1) est conçu pour détecter des signaux biologiques (19) provenant du patient (2) le long de différents vecteurs de détection (20, 20a, 20b) ;
dans lequel l'étape consistant à appliquer le mode de fonctionnement sélectionné (12) amène le dispositif médical (1) à commuter entre les vecteurs de détection (20, 20a, 20b).

5. Procédé selon l'une des revendications précédentes,
dans lequel, en appliquant le mode de fonctionnement sélectionné (12), au moins un composant matériel (18) du dispositif médical (1), en particulier au moins un capteur (18) du dispositif médical (1), est désactivé.

6. Procédé selon l'une des revendications précédentes,
dans lequel le dispositif médical (1) comprend un détecteur d'interférences (24) configuré pour détecter des interférences dans un signal de sortie (16) fourni par au moins un capteur (18) du dispositif médical (1) ;
dans lequel, en appliquant le mode de fonctionnement sélectionné (12), le détecteur d'interférences (24) est modifié ou désactivé.

7. Procédé selon l'une des revendications précédentes,
dans lequel l'étape consistant à appliquer le mode de fonctionnement sélectionné (12) empêche le dispositif médical (1) de commuter dans un mode d'économie d'énergie.

8. Procédé selon l'une des revendications précédentes,
dans lequel le dispositif médical (1) comprend une fonction d'enregistrement (26) configurée pour enregistrer un signal de sortie (16) fourni par au moins un capteur (18) du dispositif médical (1) ;
dans lequel l'étape consistant à appliquer le mode de fonctionnement sélectionné (12) amène la fonction d'enregistrement (26) à :
- démarrer l'enregistrement du signal de sortie (16) et/ou marquer de nouveaux enregistrements (28) du signal de sortie (16) comme étant enregistrés en présence du dispositif externe (4) ; ou
- arrêter l'enregistrement du signal de sortie (16).

9. Procédé selon l'une des revendications précédentes,
dans lequel le mode de fonctionnement sélectionné (12) est appliqué uniquement aussi longtemps que le signal de détection (6) est reçu.

10. Procédé selon l'une des revendications précédentes,
dans lequel le signal de détection (6) est reçu par l'intermédiaire d'une liaison de communication de données sans fil pour la communication de données, en particulier la communication de données unidirectionnelle, entre le dispositif médical (1) et le dispositif externe (4).

11. Procédé selon l'une des revendications précédentes,
dans lequel le signal de détection (6) est reçu par Bluetooth.

12. Unité de commande (7) comprenant un processeur (8) conçu pour mettre en œuvre le procédé selon l'une des revendications précédentes.

13. Dispositif médical implantable (1) comprenant l'unité de commande (7) selon la revendication 12.

14. Dispositif médical implantable (1) selon la revendication 13, dans lequel le dispositif médical implantable est au moins un parmi un générateur d'impulsions implantable (GII), un défibrillateur automatique implantable (DAI), un stimulateur pour thérapie de resynchronisation cardiaque (CRT), un neurostimulateur, un enregistreur d'électrocardiogramme (ECG), un capteur de pression, un capteur biochimique, une pompe de médicament ou un appareil auditif.

15. Programme informatique comprenant des instructions qui, lorsque le programme est exécuté par un processeur (8), amènent le processeur (8) à mettre en œuvre le procédé selon l'une quelconque des revendications 1 à 11.
